(19) Europäisches Patentamt
European Patent Office
Office européen des brevets

(11) Numéro de publication: **0 115 988**
**B1**

(12) **FASCICULE DE BREVET EUROPÉEN**

(45) Date de publication du fascicule du brevet: **15.10.86**

(51) Int Cl.4: **C 12 P 21/00**, A 61 K 37/02 // C12R1/22

(21) Numéro de dépôt: **84400172.7**

(22) Date de dépôt: **26.01.84**

(54) **Nouveau procédé de préparation d'acylglycoprotéines immunostimulantes extraites de Klebsiella pneumoniae, compositions pharmaceutiques antiallerqiques.**

(30) Priorité: **28.01.83 FR 8301345**

(43) Date de publication de la demande:
**15.08.84 Bulletin 84/33**

(45) Mention de la délivrance du brevet:
**15.10.86 Bulletin 86/42**

(84) Etats contractants désignés:
**AT BE CH DE GB IT LI LU NL SE**

(56) Documents cité:
**EP-A-0 013 851**
**EP-A-0 069 266**
**FR-A-2 396 019**
**FR-A-2 396 020**
**FR-A-2 462 477**
**FR-A-2 490 496**

(73) Titulaire: **ROUSSEL- UCLAF, 35, boulevard des Invalides, F-75007 Paris (FR)**

(72) Inventeur: **Smets, Pierre, 109, rue de L'Ourcq, F-75009 Paris (FR)**
Inventeur: **Zalisz, René, 20, rue Delattre de Tassigny, F-95180 Menucourt (FR)**

(74) Mandataire: **Fritel, Hubert, Département des Brevets ROUSSEL UCLAF B.P. no 9, F-93230 Romainville (FR)**

**EP 0 115 988 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1986

**Description**

La présente invention concerne un nouveau procédé de préparation d'acylglycoprotéines immunostimulantes extraites de Klebsiella pneumoniae et des compositions pharmaceutiques antiallergiques les renfermant.

La demande de brevet français n° 2 490 496, ainsi que la demande de brevet européen n° 0 049 182 décrivent notamment de nouvelles glycoprotéines immunostimulantes extraites de Klebsiella pneumoniae caractérisées en ce qu'elles renferment 30% à 45% de protéines, 30% à 40% d'oses neutres, moins de 4% d'acide glucuronique, 2% à 5% d'osamines, et ont un poids moléculaire d'environ 350 000 daltons. Elles décrivent plus particulièrement les protéines ci-dessus caractérisées en ce que la fraction protéique est composée par environ 30% d'acides aminés acides, en ce que la fraction polysaccharidique renferme approximativement une molécule de glucose pour quatre molécules de galactose et est essentiellement composée de la répétition de l'unité polysaccharidique dont la structure est la suivante:

$$\left[\left({}^{3}\mathrm{galactose}^{1}\right)_{m} - {}^{3}\mathrm{galactose}^{1} - {}^{4}\mathrm{glucose}^{1}\right]_{n}$$

dans laquelle m est un nombre entier égal à 3, 4 ou 5. Ces demandes décrivent également un procédé de préparation de ces glycoprotéines caractérisé en ce que l'on traite, à l'aide d'un ammonium quaternaire, une solution de glycoprotéines obtenue par diafiltration d'un extrait de lysat de cultures de Klebsiella pneumoniae, isole le surnageant correspondant par élimination du précipité obtenu, traite à froid à l'aide d'un alcanol de faible poids moléculaire le surnageant correspondant à une solution saline des glycoprotéines, obtient ainsi un nouveau précipité que l'on redissout dans l'eau, dialyse, lyophilise, remet en solution, filtre sur gel, puis recueille la première fraction éluée, concentre, et si désiré, amène à sec.

Ces demandes décrivent aussi l'activité immunostimulante desdites glycoprotéines, et leur utilisation en thérapeutique, en particulier présentées sous forme de compositions pharmaceutiques.

Les études effectuées depuis le dépôt de ces demandés ont permis de préciser la structure de ces glycoprotéines. Il s'agit bien de glycoprotéines, mais plus précisément d'acylglycoprotéines, dont la chaîne polysaccharidique est reliée à un reste asparaginé de la chaîne protéique par un core formé d'heptose et de 2-céto-3-déoxy-octulosonate, suivi d'une partie acylée comportant de l'acide β -hydroxymyristique, puis de la N-acétylglucosaminé. Ces études ont également permis de mettre en évidence l'activité antiallergique desdites glycoprotéines.

En poursuivant également ses études sur le plan du procédé, la demanderesse a découvert qu'il était possible de mieux prendre en compte les impératifs économiques liés à la mise en oeuvre d'un procédé industriel en concentrant le surnageant recueilli après élimination du précipité obtenu par action d'un sel d'ammonium quaternaire. Cette concentration est avantageusement réalisée par utilisation de dispositifs de sélection de molécules à perméabilité sélective tels que des ultrafiltres. En effet, ces dispositifs permettent d'une part de traiter des volumes moindres dans la suite du procédé, et d'autre part, de manière inattendue, de se dispenser des étapes ultérieures de dialyse et de filtration sur gel de l'art antérieur.

C'est ainsi que la présente demande a pour objet un nouveau procédé de de préparation des glycoprotéines telles que décrites et revendiquées dans la demande de brevet français n° 2 490 496, ainsi que dans la demande de brevet européen n° 0 049 182, procéde dans lequel l'on prépare par diafiltration d'un extrait de lysat de cultures de Klebsiella pneumoniae, une solution de glycoprotéines purifiées, traite ladite solution à l'aide d'un halogénure d'un ammonium quaternaire, et isole le surnageant par élimination du précipité ainsi obtenu, ledit procédé étant caractérisé en ce que l'on concentre le surnageant par utilisation d'au moins un dispositif de sélection de molécules, traite à froid le concentré à l'aide d'au moins un alcanol de faible poids moléculaire, isole le précipité ainsi obtenu, si désiré le lave à l'aide d'au moins un alcanol de faible poids moléculaire, et le sèche.

Ce nouveau procédé est étonnament plus performant que celui de l'art antérieur puisqu'il permet en particulier de se dispenser de deux étapes longues et coûteuses, ainsi que de diminuer les volumes d'alcanol manipulés.

La concentration peut se faire par les méthodes usuelles de sélection de molécules, en particulier par utilisation de membranes à perméabilité sélective, notamment des ultrafiltres.

Les membranes à perméabilité sélective peuvent être de natures diverses. Elles peuvent être en acétate de cellulose telles les membranes de type HF-U (Dow Chemicals), HF-U (Kalle Chemie), SEPA-CA (Osmonics), ou SM (Sartorius). Elles peuvent être à base de polyélectrolytes complexes, telles les membranes de type UM (Amicon) ou IRIS 3042 (Rhone-Poulenc). Elles peuvent être également à base de polysulfones, telles les membranes de type H10P (Amicon et Romicon), SEPA PS (Osmonics) ou IRIS 3022 (Rhone-Poulenc). Elles peuvent aussi être à base de polyamides telles que les membranes de type BM ou BHF (Berghof), à base de polymères aromatiques, telles les membranes de type PM, MX ou HF (Amicon et Romicon), à base de copolymères de

chlorure de vinyle et d'acrylonitrile, de polyoléfines substituées ou encore composites, à base de polysulfones sur support de polyéthylène telles les membranes de type PT (Millipore).

Ces membranes peuvent se présenter sous forme plane, tubulaire ou encore sous forme de fibres creuses ou de spirales.

Afin d'obtenir les acylglycoprotéines désirées sous forme pure, on utilise de préférence des ultrafiltres dont le seuil de coupure est calibré à un poids moléculaire pouvant aller de 5000 à 100000 daltons.

Dans des conditions préférentielles de mise en beuvre du procédé, l'on utilise des ultrafiltres se présentant sous forme de fibres creuses, notamment ceux de nature polysulfonique ayant un seuil de coupure fixé à 5 000 daltons. On utilise alors avantageusement les membranes de type H10P5 fabriquées par les sociétés Amicon et Romicon.

L'opération de concentration est avantageusement réalisée aux environs de la température ambiante, par exemple entre 20° C et 30° C. Elle peut se faire selon les techniques préconisées par le constructeur des membranes utilisées. Il est possible d'utiliser une seule membrane ou plusieurs membranes différentes, et l'opération de concentration peut être réalisée en une ou plusieurs fois, de manière continue ou discontinue.

Lorsque l'on utilise des fibres creuses, la concentration est réalisée de préférence en deux cycles de traitement en util'sant la technique "wash in" qui consiste à compenser les pertes de la chambre de filtration en petites molécules et en solvant par un apport de solvant (de l'eau dans le cas de la présente invention) dans la chambre de filtration.

La concentration qu'il est souhaitable d'obtenir est d'environ 5 fois celle de la solution de départ.

La solution obtenue est alors traitée à froid aux environs de +4°C, à l'aide d'un alcanol de faible poids moléculaire tel que le méthanol, l'éthanol, le n-propanol ou l'isopropanol.

On utilise de préférence l'éthanol.

Les résultats les plus intéressants sont obtenus par l'utilisation de six volumes d'éthanol pour un volume de solution saline, pendant une nuit à la température de +4° C.

On utilise ensuite une ou plusieurs techniques d'isolement des précipités, comme par exemple la décantation suivie de filtration, la filtration seule, ou la centrifugation.

Le précipité peut alors avantageusement être séché, à pression ambiante par exemple, mais de préférence sous pression reduite, en présence de déshydratant ou non. A titre de déshydratant, on peut par exemple utiliser la potasse, l'anhydride phosphorique ou, de préférence, le chlorure de calcium. Le séchage peut être aidé par un léger chauffage, de préférence à température inférieure à 40° C. Si désiré, le précipité peut à ce moment être homogénéisé, par exemple par broyage mécaniqué.

Le précipité peut également être dissous dans l'eau et amené à sec, par exemple par atomisation ou par lyophilisation.

La lyophilisation est alors effectuée de façon classique, par exemple dans des ensembles congélateur-sublimeur de taille moyenne comme les modèles SMU ou SMRG commercialisés par la Société Usifroid, des lyophilisateurs de grande taille comme, par exemple, l'ensemble formé par un congélateur CA1 et un sublimeur SMIRS, tous deux commercialisés par Usifroid.

Des modèles plus petits de laboratoire peuvent aussi être utilisés, ainsi que ceux commercialisés par d'autres sociétés telle la Société Sérail.

Les produits de départ du procédé, objet de la présente demande, peuvent être préparés comme indiqué dans le brevet français n° 2.171.907, c'est-à-dire par exemple par mise en culture des germes (Klebsiella pneumoniae CIP 52145) identique à la souche déposée par la demanderesse le 29 juin 1981 sous le n° I-163 à l'Institut Pasteur à Paris, lyse des germes, séchage (lyophilisation par exemple) extraction à l'aide de solvants des lipides, déprotidation physique (centrifugation par exemple), puis ultrafiltration et séchage, par exemple par lyophilisation.

Lorsqu'elles sont formulées à l'aide d'un excipient approprié, les glycoprotéines telles que décrites dans la demande de brevet français n° 2 490 496, ainsi que dans la demande de brevet européen n° 0 049 182, de même que celles décrites dans le brevet français n° 2 171 907, sont douées de remarquables propriétés antiallergiques.

C'est ainsi que la présente demande a également pour objet les glycoprotéines telles qu'obtenues dans le procédé ci-dessus décrit de même que les glycoprotéines telles qu'obtenues par le procédé décrit dans le brevet français n° 2.171.907, pour leur utilisation dans le traitement des maladies allergiques ainsi que les compositions pharmaceutiques destinées au traitement des maladies allergiques, caractérisées en ce qu'elles sont constituées des glycoprotéines telles que décrites et revendiquées dans la demande de brevet français n° 2.490.496, ainsi que dans la demande de brevet européen n° 0.049.182, de même que celles décrites dans le brevet français n° 2.171.907, ainsi que d'un excipient approprié.

Ces compositions pharmaceutiques peuvent être, par exemple, solides ou liquides et se présenter sous les formes pharmaceutiques couramment utilisées en médecine humaine, pour le traitement des maladies allergiques, comme par exemple les comprimes, simples ou dragéifiés, les gélules, les sirops, les aérosols, les suppositoires, les préparations injectables, les crèmes, les pommades; elles sont préparées selon les méthodes usuelles. Le ou les principes actifs peuvent y âtre incorporés à des excipients habituellement employés dans ces compositions pharmaceutiques, tels que le talc, la gomme arabique, le lactose, l'amidon, le stéarate de

magnésium, le beurre de cacao, les véhicules aqueux ou non, les corps gras d'origine animale ou végetale, les dérivés paraffiniques, les glycols, les divers agents mouillant, dispersants ou émulsifiants, les conservateurs, les colorants, les aromatisants.

Les glyprotéines et compositions, objet de l'invention, trouvent en particulier leur utilisation dans le traitement des allergies respiratoires telles que les rhinites allergiques ou les trachéites ou laryngites, des allergies cutanées telles que l'eczéma ou l'urticaire, des allergies oculaires ou des manifestations allergiques de diverses origines: piqûres animales ou allergies alimentaires par exemple. Les exemples qui suivent illustrent la présente invention.

**Exemple 1**

On met en solution aqueuse à 10 g/litre, à 4° C pendant 16 heures, 1 kg de produit tel qu'obtenu à l'exemple 1 du brevet françaais n° 2 171 907, préparé à partir de la souche de Klebsiella pneumoniae 52145 ou I 163 de la collection de L'Institut Pasteur. On ajoute 0-8 volume de solution à 3% de bromure de cétyltriméthylammonium à raison de 1 litre/minute environ, et agite modérément pendant 1 heure. On élimine le précipité formé par centrifugation en continu à débit de 5 litres/heure environ, à 62 000 g.

Le surnageant est concentré par ultrafiltration sur fibres creuses à seuil de rétention fixé à 5 000 (Hollow Fibers H10P5 commercialisées par Amicon et Romicon) en 2 cycles de traitement dans les proportions 5/1. On ajoute à la vitesse de 3 litres par minute, 6 volumes d'éthanol à 96% et agite modérément pendant 15 minutes. On laisse décanter, essore et rince le précipité, sèche à moins de 40° C en présence de déshydratant, homogéinise par broyage mécanique, et obtient 200 g de produit attendu possédant les caracteristiques du produit decrit dans la demande de brevet français n° 2.490.496, ainsi que dans la demande de brevet européen n° 0.049.182.

**Exemple 2**

On a préparé des comprimés répondant à la formule:
- Produit de l'exemple 1 ............................... 1 mg
- Excipient q.s. pour un comprimé terminé à ...................... 100 mg.

(Détail de l'excipient: lastose, amidon, talc, stéarate de magnésium).

**Exemple 3**

On a préparé des aérosols délivrant des doses contenant chacune:
- Produit de l'exemple 1 ...................... 0,5mg
- Emulsifiant ........................................ 0,15mg
- Propulseur ......................................... 50 mg.

**Exemple 4**

On a préparé une crème répondant à la formule:
- Produit de l'exemple 1 .............................. 1 mg
- Excipient: 2-octyl-dodécanol, alcool cétostéarylique, cétostéaryl sulfate de sodium, parahydroxybenzoate de méthyle et de propyle, eau purifiée .......................... 10 g.

**Exemple 5**

On a préparé des comprimés répondant à la formule:
- Produit de l'exemple 1 du brevet français n° 2 171 907.. 1mg
- Excipient q.s. pour un comprimé terminé à............. 100mg.

(Détail de l'excipient: lactose, amidon, talc, stéarate de magnésium).

**ACTIVITE ANTIALLERGIQUE**

Principe:

Les sujets allergiques synthétisent des anticorps (Immuno globulines E ou IGE) contre l'allergène auquel ils sont sensibles. Ces anticorps sont capables de se fixer sur les polynucléaires (ou granulocytes) basophiles de ces sujets et entraînent une modification de la membrane des polynucléaires qui provoque leur dégranulation.

Test:

La technique est inspirée de celle de Benveniste Chim. Allergy., 1981, 11, p. 1 à 11. On prépare par décantation un plasma enrichi en polynucléaires basophiles d'un sujet sensible a un allergène donné, à partir d'une partie de sang et 9 parties de colorant (May-Grünwald-Giensa). La suspension-témoin et la suspension additionnée du produit à tester sont mises en contact avec le même allergène. On évalue alors la dégranulation en pourcentage:

$$\% = \frac{\text{Nombre de basophiles de la suspension témoin} - \text{Nombre de basophiles de la suspension traitée}}{\text{Nombre de basophiles de la suspension témoin}}$$

Un agent antiallergique diminue le pourcentage de dégranulation des polynucléaires basophiles.

Résultats:

1) Allergène: poils de chat.

A la concentration de 100 µg de produit de l'exemple 1 par ml, on n'observe aucune dégranulation dans la suspension traitée, pour une dégranulation de 61% de la suspension-témoin.

2) Allergène: corps entier de moustique.

Aux concentrations de 2 µg de produit de l'exemple 1 ou de produit de départ de l'exemple 1 par ml, on n'observe aucune dégranulation dans la suspension traitée, pour une dégranulation de 60% de la suspension témoin.

**Revendications**

pour les Etats contractants BE CH DE GB IT LI LU NL SE

1) Procédé de préparation de glycoprotéines dans lequel dans l'ordre l'on prépare par dialfiltration d'un extrait de lysat de cultures de Klebsiella pneumoniae, une solution de glycoprotéines purifiées, traite ladite solution à l'aide d'un halogénure d'un ammonium quaternaire, isole le surnageant par élimination du précipité ainsi obtenu, concentre le surnageant par utilisation d'au moins un dispositif de sélection de molécules, traite à froid le concentré à l'aide d'au moins un alcanol de faible poids moléculaire isole le précipité ainsi obtenu, si désiré le lave à l'aide d'au moins un alcanol de faible poids moléculaire, et le sèche.

2) Procédé selon la revendication 1, caractérisé en ce que le dispositif de sélection de molécules comprend une ou plusieurs membranes à perméabilité sélective.

3) Procédé selon la revendication 1 ou 2, caractérisé en ce que le dispositif de sélection de molécules est constitué d'un ou plusieurs ultrafiltres.

4) Procédé selon la revendication 3, caractérisé en ce que les ultrafiltres ont un seuil de coupure calibré à un poids moléculaires pouvant aller de 5000 à 100000 daltons.

5) Procédé selon la revendication 3 ou 4, caractérisé en ce que les ultrafiltres se présentent sous la forme de fibres creuses.

6) Procédé selon la revendication 5, caractérisé en ce que les ultrafiltres ont une nature polysulfonique et sont calibrées à 5 000 daltons.

7) Application des glycoprotéines telles qu'obtenues à l'une quelconque des revendications 1 à 6 pour obtenir un médicament destiné au traitement des maladies allergiques.

8) Application des glycoprotéines telles qu'obtenues dans l'ordre par lyse de germes de Klebsiella pneumoniae, séchage, extraction a l'aide de solvants des lipides, déprotidation physique, ultrafiltration et séchage pour obtenir un médicament destiné au traitement des maladies allergiques.

**Revendications**

pour l'Etat contractant AT

1) Procédé de préparation de glycoprotéines dans lequel dans l'ordre l'on prépare par dialfiltration d'un extrait de lysat de cultures de Klebsiella pneumoniae, une solution de glycoprotéines purifiées, traite ladite solution à l'aide d'un halogenure d'un ammonium quaternaire, isole le surnageant par élimination du précipité ainsi précipité ainsi obtenu, concentre le surnageant par utilisation d'au moins un dispositif de sélection de molécules, traite à froid le concentré à l'aide d'au moins un alcanol de faible poids moléculaire isole le précipité ainsi obtenu, si désiré le lave à l'aide d'au moins un alcanol de faible poids moléculaire, et le sèche.

2) Procédé selon la revendication 1, caractérisé en ce que le dispositif de sélection de molécules comprend une ou plusieurs membranes à perméabilité sélective.

3) Procédé selon la revendication 1 ou 2, caractérisé en ce que le dispositif de sélection de molécules est constitué d'un ou plusieurs ultrafiltres.

4) Procédé selon la revendication 3, caractérisé en ce que les ultrafiltres ont un seuil de coupure calibré à un poids moléculaires pouvant aller de 5000 à 100000 daltons.

5) Procédé selon la revendication 3 ou 4, caractérisé en ce que les ultrafiltres se présentent sous la forme de fibres creuses.

6) Procédé selon la revendication 5, caractérisé en ce que les ultrafiltres ont une nature polysulfonique et sont calibrées à 5000 daltons.

**Patentansprüche**

für die Vertragsstaaten: BE, CH, DE, GB, IT, LI, LU, NL, SE

1. Verfahren zur Herstellung von Glykoproteinen, bei dem man der Reihenfolge nach durch Diafiltration eines Extrakts des Lysats von Klebsiella pneumoniae-Kulturen eine Lösung gereinigter Glykoproteine herstellt, diese Lösung mit einem quaternären Ammoniumhalogenid behandelt, den Überstand durch Entfernung des so erhaltenen Niederschlags isoliert, den

Überstand durch Verwendung von zumindest einer Vorrichtung für die Selektionierung von Molekülen einengt, das Konzentrat mit zumindest einem Alkanol mit niedrigem Molekulargewicht in der Kälte behandelt, den so erhaltenen Niederschlag isoliert, ihn gewünschtenfalls mit zumindest einem Alkanol mit niedrigem Molekulargewicht wäscht und ihn trocknet.

2. Verfahren gemäß Anspruch 1, dadurch gekennzeichnet, daß die Vorrichtung zur Selektionierung von Molekülen eine oder mehrere Membranen mit einer selektiven Permeabilität umfaßt.

3. Verfahren gemäß Anspruch 1 oder 2, dadurch gekennzeichnet, daß die Vorrichtung für die Selektionierung von Molekülen aus einem oder mehreren Ultrafiltern besteht.

4. Verfahren gemäß Anspruch 3, dadurch gekennzeichnet, daß die Ultrafilter eine Ausschlußgrenze besitzen, die auf ein Molekulargewicht von 5000 bis 100000 Dalton eingestellt ist.

5. Verfahren gemäß Anspruch 3 oder 4, dadurch gekennzeichnet, daß die Ultrafilter in Form von Hohlfasern vorliegen.

6. Verfahren gemäß Anspruch 5, dadurch gekennzeichnet, daß die Ultrafilter vom Polysulfon-Typ sind und auf 5000 Dalton eingestellt sind.

7. Verwendung der Glykoproteine, erhalten gemäß einem der Ansprüche 1 bis 6, zur Erzielung eines Arzneimittels für die Behandlung allergischer Erkrankungen.

8. Verwendung der Glykoproteine, erhalten der Reihenfolge nach durch Lyse von Klebsiella pneumoniae-Stämmen, Trocknen, Extraktion mit Lösungsmitteln für Lipide bzw. Lipoide, physikalische Proteinentfernung, Ultrafiltration und Trocknung, zur Erzielung eines Arzneimittels für die Behandlung allergischer Erkrankungen.

**Patentansprüche**

für den Vertragsstaat: AT

1. Verfahren zur Herstellung von Glykoproteinen, bei dem man der Reihenfolge nach durch Diafiltration eines Extrakts des Lysats von Klebsiella pneumoniae-Kulturen eine Lösung gereinigter Glykoproteine herstellt, diese Lösung mit einem quaternären Ammoniumhalogenid behandelt, den Überstand durch Entfernung des so erhaltenen Niederschlags isoliert, den Überstand durch Verwendung von zumindest einer Vorrichtung zur Selektionierung von Molekülen einengt, das Konzentrat in der Kälte mit zumindest einem Alkanol mit niedrigem Molekulargewicht behandelt, den so erhaltenen Niederschlag isoliert, ihn gewünschtenfalls mit zumindest einem Alkanol mit niedrigem Molekulargewicht wäscht und ihn trocknet.

2. Verfahren gemäß Anspruch 1, dadurch gekennzeichnet, daß die Vorrichtung zur Selektionierung von Molekülen eine oder mehrere Membranen mit selektiver Permeabilität umfaßt.

3. Verfahren gemäß Anspruch 1 oder 2, dadurch gekennzeichnet, daß die Vorrichtung zur Selektionierung von Molekülen aus einem oder mehreren Ultrafiltern besteht.

4. Verfahren gemäß Anspruch 3, dadurch gekennzeichnet, daß die Ultrafilter eine Ausschlußgrenze besitzen, die auf ein Molekulargewicht von 5000 bis 100000 Dalton eingestellt ist.

5. Verfahren gemäß Anspruch 3 oder 4, dadurch gekennzeichnet, daß die Ultrafilter in Form von Hohlfasern vorliegen.

6. Verfahren gemäß Anspruch 5, dadurch gekennzeichnet, daß die Ultrafilter vom Polysulfon-Typ sind und für 5000 Dalton eingestellt sind.

**Claims**

for the Contracting States BE CH DE GB IT LI LU NL SE

1) Preparation process for glycoproteins in which, in order, a solution of purified glycoproteins is prepared by diafiltration of an extract of a lysate of cultures of Klebsiella pneumoniae, the said solution is treated with a halide of a quaternary ammonium, the supernatant is isolated by eliminating the precipitate so obtained, the supernatant is concentrated by use of at least one means of selection of molecules, the concentrate is treated cold with at least one alkanol of low molecular weight, the precipitate so obtained is isolated and, if desired, is washed with at least one alkanol of low molecular weight and dried.

2) Process according to claim 1, characterized in that the means of selection of molecules comprises one or more membranes of selective permeability.

3) Process according to claim 1 or 2, characterized in that the means of selection of molecules is constituted by one or more ultrafilters.

4) Process according to claim 3, characterized in that the ultrafilters have a cut-off threshold calibrated to a molecular weight ranging from 5,000 to 100,000 daltons.

5) Process according to claim 3 or 4, characterized in that the ultrafilters are presented in the form of hollow fibres.

6) Process according to claim 5, characterized in that the ultrafilters are of polysulphonic nature and are calibrated to 5,000 daltons.

7) The use of glycoproteins such as those obtained in any one of the claims 1 to 6 so as to obtain a medicament intended for the treatment of allergic diseases.

8) The use of glycoproteins such as those obtained, in order, by lysing germs of Klebsiella pneumoniae, drying, extracting lipids by means of solvents, physically deproteinising, ultrafiltering and drying so as to obtain a medicament intended for the treatment of allergic diseases.

## Claims

for the Contracting State AT

1) Preparation process for glycoproteins in which, in order, a solution of purified glycoproteins is prepared by diafiltration of an extract of a lysate of cultures of Klebsiella pneumoniae, the said solution is treated with a halide of a quaternary ammonium, the supernatant is isolated by eliminating the precipitate so obtained, the supernatant is concentrated by use of at least one means of selection of molecules, the concentrate is treated cold with at least one alkanol of low molecular weight, the precipitate so obtained is isolated and, if desired, is washed with at least one alkanol of low molecular weight and dried.

2) Process according to claim 1, characterized in that the means of selection of molecules comprises one or more membranes of selective permeability.

3) Process according to claim 1 or 2, characterized in that the means of selection of molecules is constituted by one or more ultrafilters.

4) Process according to claim 3, characterized in that the ultrafilters have a cut-off threshold calibrated to a molecular weight ranging from 5,000 to 100,000 daltons.

5) Process according to claim 3 or 4, characterized in that the ultrafilters are presented in the form of hollow fibres.

6) Process according to claim 5, characterized in that the ultrafilters are of polysulphonic nature and are calibrated to 5,000 daltons.